# EUROPEAN PATENT APPLICATION

(11) **EP 4 707 324 A1**
(43) Date of publication of application: **11.03.2026**
(21) Application number: 24199540.6
(22) Date of filing: 10.09.2024
(51) Int. Cl.: C08J 5/18, A61K 9/14, A61K 9/20, A61K 9/48, B32B 23/00, B32B 27/36, C08L 67/04

(54) **COMPOSTABLE PRIMARY PACKAGING FOR WRAPPING INDIVIDUAL SOLID DOSAGE UNITS OF A MEDICINAL PRODUCT**

(71) Applicant: Redcarbonpharm Limited, Weston-Super-Mare BS23 4TN (GB)
(72) Inventor: Glenny, Susan Marie, Gerrards Cross, SL9 8EY (GB)
(74) Representative: EIP

(57) **Abstract**

The present invention relates to a compostable primary packaging suitable for wrapping individual solid dosage units of a medicinal product, comprising:
a primary packaging film or laminate comprising a biodegradable and compostable polymer, the film or laminate being configured to be processed in a film wrapping machine to encase an individual solid dosage unit of a medicinal product;
the primary packaging film or laminate having sufficient mechanical strength, flexibility, and sealability to maintain an airtight and moisture-resistant barrier around the solid dosage unit after a film wrapping process;
the primary packaging film or laminate being characterized by its ability to fully decompose into non-toxic organic components within an industrial composting environment within a typical composting cycle of 90 days.

## Description

### Technical field

The present invention relates to the field of medicinal product packaging, specifically to compostable primary packaging materials designed for the wrapping of individual solid dosage units of medicinal products.

### Background

In the pharmaceutical industry, solid dosage unit packaging plays a critical role in ensuring the accurate delivery of medication, maintaining drug stability, and improving patient compliance. A solid dosage unit of a medicinal product refers to a single, pre-measured quantity of medication intended for one administration or use. Examples of solid dosage units include, but are not limited to, tablets, capsules, pills, powders and granules, and suppositories. Each solid dosage unit is typically packaged separately to maintain the stability of the drug and to provide convenience for both patients and healthcare providers.

Traditionally, such packaging has been made from non-biodegradable materials, including various plastics and aluminium foils, which are effective at protecting medicinal products but contribute significantly to environmental pollution. The widespread use of these materials has led to growing concerns about their environmental impact, particularly due to their persistence in the environment and the challenges associated with their disposal. Recycling, when available, contributes to carbon emissions due to the energy-intensive nature of the process itself. Blister packs, commonly used for pharmaceutical products, are particularly challenging to recycle because of their multi-component design, combining plastic and aluminium, which complicates separation and reprocessing. The regulatory landscape for drug packaging is highly complex, with stringent requirements enforced by agencies such as the Medicines and Healthcare products Regulatory Agency (MHRA) in the UK and the European Medicines Agency (EMA) in the EU. For example, the European Medicines Agency provides a "Guideline on Plastic Immediate Packaging Materials", adopted by the committee for medicinal products for human use (CHMP) and the committee for medicinal products for veterinary use (CVMP), which concerns the application of Part 1, Module 3, sections 3.2.1.6, 3.2.2.2 and 3.2.2.7 of Annex I to Directive 2003/63/EC, amending Directive 2001/83/EC for human medicinal products, and Part 2, sections A, C and G of Annex 1 to Directive 2001/82/EC for veterinary medicinal products, respectively, to plastic immediate packaging materials. The guideline covers the specific requirements for plastic immediate packaging materials. The data to be provided for plastic packaging materials depend, e.g., on the physical state of the active substance and the pharmaceutical dosage form.

Current manufacturers face limited incentives to adopt alternative packaging solutions due to the high costs, potential profit loss, and the need for extensive and costly trials to meet regulatory standards.

With increasing global awareness of environmental sustainability, there is a pressing need for alternative packaging solutions that can reduce the ecological footprint of medicinal products. Compostable packaging materials have emerged as a promising solution, offering the potential to break down naturally into non-toxic organic components after use, thus mitigating the long-term environmental impact associated with traditional packaging materials.

However, developing compostable packaging for medicinal products presents unique challenges. The packaging must provide sufficient mechanical strength, flexibility, and sealability to protect the medicinal product from environmental factors such as moisture, oxygen, and light. Additionally, it must ensure the stability and efficacy of the medicinal product throughout its shelf life. Furthermore, the packaging material should preferably be capable of being processed using conventional packaging machinery, and must comply with stringent compostability standards, such as those outlined in European Standard EN 13432, American Standard ASTM D6400, and International Standard ISO 17088.

### Description of the invention

An object of the present disclosure is to provide a compostable primary packaging suitable for wrapping individual solid dosage units of a medicinal product, which can replace traditional primary packaging, such as blister packs, strip packs, sachets and pouches, made from non-biodegradable materials.

Another object of the present disclosure is to provide a compostable primary packaging suitable for wrapping individual solid dosage units of a medicinal product, which is tamper proof.

The present disclosure addresses these objects by providing a primary packaging film or laminate specifically designed for wrapping individual solid dosage units of medicinal products. The primary packaging film or laminate comprises a biodegradable and compostable polymer, for example polylactic acid (PLA), polybutylene succinate (PBS), polyhydroxyalkanoates (PHA), polycaprolactone (PCL), thermoplastic starch (TPS), cellulose-based polymers, chitosan, alginates, or combinations thereof. These materials are engineered to offer the necessary barrier properties and mechanical strength to protect the medicinal product while being fully compostable under industrial composting conditions within a typical cycle of 90 days.

This innovative packaging solution offers a significant advancement in the field of medicinal product packaging, providing an environmentally friendly alternative to traditional materials without compromising the safety or effectiveness of the medicinal product. By using compostable materials that meet established standards for biodegradability and compostability, this invention contributes to the reduction of environmental pollution and supports the pharmaceutical industry's efforts to adopt more sustainable practices.

According to a first aspect illustrated herein, there is provided a compostable primary packaging suitable for wrapping individual solid dosage units of a medicinal product, comprising:
a primary packaging film or laminate comprising a biodegradable and compostable polymer, the film or laminate being configured to be processed in a film wrapping machine to encase an individual solid dosage unit of a medicinal product;
the primary packaging film or laminate having sufficient mechanical strength, flexibility, and sealability to maintain an airtight and moisture-resistant barrier around the solid dosage unit after a film wrapping process;
the primary packaging film or laminate being characterized by its ability to fully decompose into non-toxic organic components within an industrial composting environment within a typical composting cycle of 90 days.

The term primary packaging as used herein refers to the packaging intended to be in direct contact with the medicinal product. Primary packaging may also sometimes be referred to as "immediate packaging". The primary packaging is designed to protect the solid dosage units from environmental factors such as moisture, oxygen, light, and physical damage while ensuring ease of use and patient compliance. Common primary packaging types used for wrapping individual solid dosage units such as tablets, capsules, pills, and similar forms, include blister packs, strip packs, sachets and pouches, and stick packs. The choice of packaging depends on the specific requirements of the dosage form, including stability, shelf life, and administration method.

The primary packaging of the present disclosure is suitable for wrapping individual solid dosage units of a medicinal product. The term solid dosage unit is used herein as a common name to refer to various solid forms of medication that are intended to be taken individually, such as tablets, pills, capsules, and similar products. The compostable primary packaging is preferably used for solid dosage units, since the risk of content/container interaction is low and generally does not require a content/container interaction study. For non-solid and liquid dosage forms, the risk of interaction will typically require comprehensive and suitable studies specific for each product.

Preferably, the primary packaging of the present disclosure complies with existing regulatory frameworks for medicinal product packaging. Specifically, the primary packaging of the present disclosure preferably complies with the European Medicines Agency "Guideline on Plastic Immediate Packaging Materials", adopted by the committee for medicinal products for human use (CHMP) and the committee for medicinal products for veterinary use (CVMP) effective as of 1 December 2005. The data to be provided for plastic packaging materials depend, e.g., on the physical state of the active substance and the pharmaceutical dosage form.

Common types of solid dosage units of medicinal products include:
- Tablets, including compressed tablets, coated tablets, effervescent tablets, chewable tablets, buccal tablets, sublingual tablets, orally disintegrating tablets (ODTs), and extended-release tablets.
- Capsules, including hard gelatin capsules, soft gelatin capsules, modified-release capsules, and enteric-coated capsules.
- Pills, including traditional pills, which refer to small, round, solid dosage units made by rolling powdered ingredients with a binding agent.
- Lozenges and troches, including lozenges, which are solid dosage forms that dissolve slowly in the mouth to deliver medication locally or systemically, and troches, which are similar to lozenges but typically softer and designed for slow dissolution in the mouth.
- Powders and granules, including powders, which are finely divided solid dosage forms that can be taken directly or dissolved in water before consumption, and granules, which are agglomerated powders that can be swallowed whole, chewed, or dissolved in liquid.
- Pellets, including small, spherical solid dosage units often used in controlled-release formulations or for implantation.
- Suppositories, including rectal suppositories, which are solid dosage units designed for rectal administration where they dissolve or melt to deliver medication, and vaginal suppositories (pessaries), which are solid dosage units designed for vaginal administration used for local or systemic effects.
- Implants, including small, solid dosage units that are surgically placed under the skin to deliver medication over an extended period.
- Pastilles, including chewable, gummy-like solid dosage units similar to lozenges, designed for slow dissolution in the mouth.
- Caplets, including tablets shaped like capsules, designed to be easier to swallow than traditional round tablets.

The primary packaging film or laminate is characterized by its ability to fully decompose into non-toxic organic components within an industrial composting environment within a typical composting cycle of 90 days. The composting environment and cycle are based on standardized conditions, typically governed by international and national standards for compostable materials. These standards define specific criteria that materials must meet to be considered compostable under industrial composting conditions.

In some embodiments, the primary packaging film or laminate is characterized by its ability to fully decompose into non-toxic organic components within an industrial composting environment within a typical composting cycle of 90 days, in accordance with European Standard EN 13432, American Standard ASTM D6400, or International Standard ISO 17088.

EN 13432 specifies the requirements for packaging recoverable through composting and biodegradation. It includes criteria for biodegradability, disintegration, and eco-toxicity. According to EN 13432, compostable materials should degrade within 90 days under industrial composting conditions, which typically involve temperatures between 55 °C and 60 °C, along with controlled humidity and oxygen levels.

ASTM D6400 specifies the criteria for compostable plastics, requiring them to break down into carbon dioxide, water, inorganic compounds, and biomass at a rate consistent with other known compostable materials under aerobic composting conditions. Like EN 13432, ASTM D6400 requires that the material should degrade within 90 days under industrial composting conditions.

ISO 17088 provides the requirements and procedures for determining the compostability of plastics, similar to EN 13432 and ASTM D6400, emphasizing the decomposition of the material within a defined timeframe under specific composting conditions.

Industrial composting conditions typically involve a temperature between 55 °C and 60 °C, controlled humidity to optimize microbial activity, sufficient aeration or oxygen levels to support aerobic decomposition, and the material should fully disintegrate within 90 days, with the biodegradation process completing within six months, leaving no toxic residues.

The compostable primary packaging of the present disclosure comprises a primary packaging film or laminate comprising a biodegradable and compostable polymer, the film or laminate being configured to be processed in a film wrapping machine to encase an individual solid dosage unit of a medicinal product.

The primary packaging film or laminate is the main component of the compostable primary packaging, and in some embodiments, the compostable primary packaging is made entirely of the primary packaging film or laminate.

The primary packaging film or laminate is configured to be processed in a film wrapping machine to encase an individual solid dosage unit of a medicinal product. The primary packaging film or laminate is engineered to possess properties that allow it to be effectively processed through standard or specialized film wrapping machines. These machines are typically employed in the packaging of individual solid dosage forms, such as tablets, capsules, or similar medicinal products. The film or laminate material must exhibit sufficient flexibility, durability, and barrier properties to ensure the integrity and protection of the medicinal product throughout its shelf life, while also being fully compostable after use.

These requirements are fulfilled by the use in the primary packaging film or laminate of a biodegradable and compostable polymer. The biodegradable and compostable polymer is preferably a thermoplastic polymer, allowing for the primary packaging film to be processed, formed, shaped, and/or sealed using a film wrapping machine. Examples of biodegradable and compostable thermoplastic polymers that may be used in the primary packaging film or laminate include, but are not limited to polylactic acid (PLA), polyhydroxyalkanoates (PHA), polybutylene succinate (PBS), polybutylene adipate terephthalate (PBAT), polycaprolactone (PCL), thermoplastic starch (TPS), cellulose acetate, poly(lactic-co-glycolic acid) (PLGA), polybutylene fumarate (PBF), and polyvinyl alcohol (PVA/PVOH).

The primary packaging film or laminate has sufficient mechanical strength, flexibility, and sealability to maintain an airtight and moisture-resistant barrier around the solid dosage unit after a film wrapping process.

The primary packaging film or laminate is preferably sealable by applying heat and/or pressure. Examples of sealing techniques include, but are not limited to, heat sealing, crimping, or ultrasonic sealing. In other words, the primary packaging film or laminate comprises a material or layer configured or arranged to allow sealing of the compostable primary packaging around the solid dosage unit by applying heat and/or pressure. The material or layer configured or arranged to allow sealing of the compostable primary packaging around the solid dosage unit by applying heat and/or pressure may for example be the film or laminate itself, or an outer layer of the laminate, or a coating layer.

In some embodiments, the biodegradable and compostable polymer is selected from the group consisting of polylactic acid (PLA), polybutylene succinate (PBS), polyhydroxyalkanoates (PHA), polycaprolactone (PCL), thermoplastic starch (TPS), cellulose-based polymers, chitosan, alginates, or a combination thereof.

In some embodiments, the biodegradable and compostable polymer is selected from the group consisting of polylactic acid (PLA), polybutylene succinate (PBS), polyhydroxyalkanoates (PHA), polycaprolactone (PCL), thermoplastic starch (TPS), or a combination thereof.

In some embodiments, the biodegradable and compostable polymer is polylactic acid (PLA). PLA is a biodegradable and compostable polymer derived from renewable resources like corn starch or sugarcane. It has good mechanical properties, including rigidity and clarity, which make it suitable for forming packaging films. PLA also offers moisture and oxygen barrier properties, useful for protecting solid dosage units of medicinal products.

In some embodiments, the biodegradable and compostable polymer comprises polybutylene succinate (PBS). PBS is a biodegradable aliphatic polyester known for its flexibility, toughness, and heat resistance. It decomposes under composting conditions and is often used in packaging films for its durability and ease of processing. Its mechanical properties make it a good choice for applications requiring more flexibility and impact resistance.

In some embodiments, the biodegradable and compostable polymer comprises polyhydroxyalkanoate (PHA). PHAs are a family of biodegradable polyesters produced by microbial fermentation of renewable resources. They exhibit excellent biodegradability in various environments, including soil and marine settings, making them highly suitable for sustainable packaging. PHA also provides good barrier properties against moisture and oxygen, which are useful for protecting medicinal products.

In some embodiments, the biodegradable and compostable polymer comprises polycaprolactone (PCL). PCL is a biodegradable polyester with a relatively low melting point, offering excellent flexibility and good compatibility with other biopolymers. It degrades in composting environments and is often used as a softening agent in blends with other polymers. Its flexibility and toughness make it useful for packaging films for medicinal products.

In some embodiments, the biodegradable and compostable polymer comprises a thermoplastic starch (TPS). TPS is derived from natural starch and plasticized with water or glycerol to create a biodegradable and compostable material. It is a sustainable, renewable resource that provides good barrier properties when blended with other biopolymers. TPS is particularly useful in packaging applications where biodegradability and cost-effectiveness are prioritized.

A combination of these polymers, such as blending PLA with PBS or PCL, can enhance the overall performance of the primary packaging film or laminate by balancing rigidity, flexibility, and barrier properties. Such blends can create films with improved mechanical strength and better protection for solid dosage units, while still being fully compostable and biodegradable.

In some embodiments, the biodegradable and compostable polymer comprises an algae-based polymer. Algae-based biodegradable and compostable polymers offer several advantages that make them particularly suitable for use in compostable packaging films for medicinal products. Algae are a fast-growing, renewable resource that can be cultivated with minimal environmental impact, requiring less land and water than traditional crops. This makes algae-based polymers an eco-friendly alternative to petroleum-based plastics, aligning with sustainability goals in pharmaceutical packaging. Algae-based polymers are naturally biodegradable and compostable, breaking down into non-toxic byproducts in composting environments. This is crucial for reducing the environmental footprint of medicinal packaging waste, which typically requires strict disposal due to potential contamination. Algae-based polymers are inherently non-toxic, making them safe for use in packaging medicinal products where safety and contamination risk are concerns. Their biocompatibility ensures they won't interact chemically with the medication, preserving product integrity. Algae-based materials can offer excellent barrier properties, such as moisture and oxygen resistance, which are essential for maintaining the stability and efficacy of solid dosage medicinal products. Effective barrier protection ensures that the medicine is shielded from environmental factors that could degrade its quality. Algae-based polymers can be engineered or blended with other biodegradable materials to tailor mechanical strength, flexibility, and barrier performance. This allows the packaging to meet specific requirements, such as protecting the medicinal product from moisture, oxygen, or light. Examples of algae-based biodegradable and compostable polymers include alginate (alginic acid), agar, and carrageenan.

Alginate is a natural polysaccharide extracted from brown algae. It is biodegradable, compostable, and offers good film-forming properties. Alginate has excellent moisture barrier capabilities, making it suitable for protecting medicinal products from humidity. It can also be used as a coating or combined with other biopolymers for enhanced barrier performance.

Agar is another algae-derived polysaccharide, primarily extracted from red algae. It is biodegradable, compostable, and has gel-like properties, making it useful in film-forming applications. Agar-based films are known for their excellent oxygen barrier properties, which are essential for protecting medicinal products from oxidation.

Carrageenan is a biodegradable polysaccharide extracted from red seaweed with film-forming abilities. Carrageenan-based films are compostable and can offer good moisture and oxygen barrier properties. It is often used in combination with other polymers to enhance mechanical strength and flexibility in packaging films.

In some embodiments, the primary packaging film or laminate comprises a laminate (also referred to as a multi-layer structure), wherein at least one layer is composed of a biodegradable and compostable polymer selected from the group consisting of polylactic acid (PLA), polybutylene succinate (PBS), polyhydroxyalkanoates (PHA), polycaprolactone (PCL), thermoplastic starch (TPS), or a combination thereof.

Different layers of the laminate may be composed of different biodegradable and compostable polymers, wherein each layer is designed to contributes to the overall barrier properties, mechanical strength, and sealability of the laminate.

In some embodiments, the laminate comprises a first layer composed of polylactic acid (PLA) to provide strength and rigidity, and a second layer composed of polybutylene succinate (PBS) to provide flexibility and sealability.

In some embodiments, the laminate comprises an inner layer composed of polyhydroxyalkanoate (PHA) for direct contact with the medicinal product, and an outer layer composed of a thermoplastic starch (TPS) for improved barrier properties against moisture and oxygen.

In some embodiments, the laminate comprises a layer of polycaprolactone (PCL) to provide heat-sealability, and a layer of polylactic acid (PLA) to provide strength and rigidity.

In some embodiments, the laminate comprises a layer of comprising, or consisting of, an algae-based polymer.

In some embodiments, the laminate comprises a layer of a biodegradable adhesive, bonding the biodegradable and compostable polymer layers of the laminate, while ensuring that the entire laminate structure remains fully compostable.

In some embodiments, the primary packaging film or laminate comprises a coextruded film of polylactic acid (PLA) and polybutylene succinate (PBS), offering a balance of mechanical strength, flexibility, and compostability.

In some embodiments, the primary packaging film or laminate further comprises an outer surface treatment or coating to, for example to enhance barrier properties against oxygen and moisture, or sealing properties, or both. In some embodiments, the primary packaging film or laminate comprises an outer surface biodegradable and compostable polymer coating. Examples of biodegradable and compostable thermoplastic polymers that may be used in the polymer coating to enhance barrier properties against oxygen and moisture, or sealing properties, or both, include, but are not limited to polylactic acid (PLA), polyhydroxyalkanoates (PHA), cellulose nanocrystals (CNC), cellulose nanofibers (CNF), chitosan, starch-based coatings, polyvinyl alcohol (PVA/PVOH), carnauba wax, beeswax, alginates, silk protein (fibroin), and lignin.

In some embodiments, the primary packaging film or laminate further comprises a thin-film coating layer to enhance barrier properties against oxygen and moisture. The term "thin-film coating" refers to a very fine layer of material, typically ranging in thickness from a few nanometers to several micrometers, which is applied to a substrate surface to modify its physical, chemical, or functional properties. Thin-film coatings are commonly used to provide barriers (e.g., against moisture, oxygen, or UV light), enhance surface durability, or add other protective or decorative functions without significantly altering the bulk properties of the substrate. These coatings can be applied using various techniques, including vacuum deposition, spraying, and dipping. Due to their low thickness, thin-film coatings typically have a low impact on the compostability of the coated film. Examples of biodegradable and compostable thermoplastic polymers that may be used in the polymer coating to enhance barrier properties against oxygen and moisture include, but are not limited to silicon dioxide (SiO₂), calcium carbonate (CaCO₃), zinc oxide (ZnO), magnesium oxide (MgO), chitosan, lignin, cellulose-based coatings (e.g., regenerated cellulose). Although not compostable, the primary packaging film or laminate further comprises a very thin metallization coating, e.g. an aluminium coating applied by vacuum coating methods. While traditional metallization can hinder compostability, very thin metal layers having a thickness in the range of 10 to 100 nm on compostable substrates can sometimes remain compostable under industrial conditions. These films provide excellent gas and moisture barriers.

In some embodiments, the primary packaging film or laminate comprises at least 50 wt%, at least 60 wt%, at least 70 wt%, at least 80 wt%, or at least 90 wt% of the biodegradable and compostable polymer, based on the total weight of the primary packaging film or laminate.

In some embodiments, the primary packaging film or laminate further comprises up to 30 wt% based on the total weight of the primary packaging film or laminate, of a biodegradable and compostable material that can be used in combination with the thermoplastic biodegradable and compostable polymer to enhance the functional properties of the primary packaging films or laminate, such as strength, flexibility, and barrier performance, while ensuring environmental sustainability. Examples of such biodegradable and compostable materials include, but are not limited to cellulose, starch, chitosan, alginates, lignin, proteins, (e.g., soy protein, zein), wax (e.g., beeswax, carnauba wax), polyvinyl alcohol (PVA/PVOH), natural fibers (e.g., hemp, flax, bamboo).

In some embodiments, the primary packaging film or laminate further comprises up to 20 % by weight of compostable additives, for example selected from the group consisting of plasticizers, compatibilizers, nucleating agents, antioxidants, antimicrobial agents, fillers, barrier enhancers, colorants, UV stabilizers, or biodegradable adhesives.

In some embodiments, the primary packaging film or laminate is free from non-compostable additives.

The suitable thickness or grammage of the primary packaging film or laminate for hermetically sealing and protecting a solid dosage unit of a medicinal product would depend on the specific material properties and the required barrier performance against moisture, oxygen, and physical damage. Higher thickness or grammage is often necessary to achieve excellent barrier properties, especially for products that are sensitive to moisture or oxygen. A thicker film or laminate provides greater resistance to tearing and puncturing during handling and transportation. The film or laminate should have sufficient thickness to form a reliable, hermetic seal during the heat-sealing process.

For single-layer films the film should preferably have a thickness in the range of 50 to 100 microns (µm). Thicker films, around 80 to 100 µm, may be used when additional mechanical strength and barrier protection are needed, especially for moisture- or oxygen-sensitive products.

For laminates (multi-layer structures) the laminate should preferably have a thickness in the range of 80 to 150 microns (µm), depending on the number of layers and the desired barrier properties. Each layer can contribute specific properties such as heat-sealability, mechanical strength, or barrier performance.

The grammage of the film or laminate may preferably be in the range of from 40 to 120 grams per square meter (g/m²). For more sensitive medicinal products requiring higher protection, grammages closer to 100-120 g/m² are preferred. Laminates typically have a higher grammage due to the additional layers providing extra protection.

The compostable primary packaging should preferably remain structurally and functionally stable for a period of time which exceeds the shelf life of the solid dosage unit of the medicinal product. This stability ensures that the packaging maintains its protective properties, such as moisture and oxygen barriers, mechanical integrity, and chemical inertness, throughout the intended storage period of the medicinal product. Stability is important to protect the medicinal product from environmental factors that could degrade its potency or safety. The packaging resists degradation, permeability, and breakdown caused by factors such as humidity, temperature, or light exposure during storage and transportation. Only after the product is consumed or expired should the packaging begin its biodegradation or composting process, ensuring both product safety and environmental sustainability. In order to achieve this, the compostable packaging materials are formulated to meet the stability requirements of pharmaceutical packaging, balancing biodegradability with extended durability during the lifecycle of the product.

In some embodiments the compostable primary packaging remains structurally and functionally stable for a period of at least 2 years. In some embodiments, the compostable primary packaging remains structurally and functionally stable for a period of at least 3 years. In some embodiments, the compostable primary packaging remains structurally and functionally stable for a period of at least 4 years. Stability may for example be evaluated at a temperature in the range of 10-40 °C, such as in the range of 15-25 °C, and a relative humidity in the range of 30-50%.

The compostable primary packaging for wrapping individual solid dosage units of a medicinal product can conveniently be realized by using a using a compostable primary packaging film or laminate in an automated film wrapping process. The film wrapping process may preferably be a wrapping process of the form-fill-seal (FFS) type. FFS packaging is a general term for a packaging process where the packaging material is formed into a specific shape (such as a bag, pouch, or wrapper), filled with the product, and then sealed. FFS can be carried out in either a horizontal (HFFS) or vertical (VFFS) orientation, depending on the product and packaging requirements.

Horizontal Form-Fill-Seal (HFFS): In HFFS packaging, the packaging material is formed into a horizontal tube, the product is filled into the tube, and then the package is sealed.

Flow wrapping is a common type of HFFS where the packaging material (often a flexible film) wraps around the product in a continuous horizontal motion, and the film is sealed around the product as it moves through the machine, creating a hermetically sealed package.

According to a second aspect illustrated herein, there is provided a method for wrapping individual solid dosage units of a medicinal product using a compostable primary packaging film or laminate, comprising the steps of:
a) providing a compostable primary packaging comprising a primary packaging film or laminate according to any one of the preceding claims as described above with reference to the first aspect;
b) feeding the primary packaging film or laminate into a film wrapping machine, wherein the film or laminate is configured to be formed around the individual solid dosage units of the medicinal product;
c) placing individual solid dosage units of the medicinal product onto the primary packaging film or laminate as it moves through the film wrapping machine; and
d) sealing the film or laminate around each individual solid dosage unit by applying heat and/or pressure to create a hermetic seal, thereby ensuring an airtight and moisture-resistant barrier.

In some embodiments of the second aspect illustrated herein, there is provided a method for wrapping individual solid dosage units of a medicinal product using a compostable primary packaging film or laminate, comprising the steps of:
a) providing a compostable primary packaging comprising a primary packaging film or laminate according to any one of the preceding claims as described above with reference to the first aspect;
b) feeding the primary packaging film or laminate into a flow wrapping machine, wherein the film or laminate is configured to be formed into a continuous tubular shape around the individual solid dosage units of the medicinal product;
c) placing individual solid dosage units of the medicinal product onto the primary packaging film or laminate as it moves through the flow wrapping machine;
d) sealing the film or laminate around each individual solid dosage unit by applying heat and/or pressure to create a hermetic seal, thereby ensuring an airtight and moisture-resistant barrier; and optionally
e) cutting the continuous tubular film into individual packages, each containing an individual solid dosage unit of the medicinal product; and
f) collecting the individually wrapped solid dosage units for further handling, storage, or distribution.

An embodiment of a flow wrapping method for wrapping individual solid dosage units of a medicinal product will now be described in more detail.

In step a) a compostable primary packaging comprising a primary packaging film or laminate according to any one of the preceding claims as described above with reference to the first aspect is provided. The primary packaging film or laminate is selected for its barrier properties, providing protection against moisture, oxygen, and light, while also being compatible with the wrapping process. The primary packaging film or laminate may be a single-layer or multi-layer structure, wherein each layer contributes to the barrier properties, mechanical strength, and sealability. In the case of laminates, one layer may serve as a heat-sealing layer, while others contribute to the overall integrity and protection of the medicinal product.

In step b) the primary packaging film or laminate is fed into a flow wrapping machine, specifically designed for processing compostable films. The film or laminate is configured to be transformed into a continuous tubular shape as it passes through the machine. This is achieved by guiding the film or laminate around a forming collar, which shapes the flat material into a cylindrical form. The speed and tension of the film are carefully controlled to ensure smooth feeding and to prevent material tearing or wrinkling, which is particularly important when dealing with compostable materials that may have different mechanical properties compared to conventional plastics.

The flow wrapping machine is equipped with rollers, guide tracks, and tension control systems to ensure the film or laminate is accurately aligned and consistently processed throughout the wrapping cycle.

In step c), the individual solid dosage units (such as tablets, capsules, or other medicinal forms) of the medicinal product are placed onto the primary packaging film or laminate as the primary packaging film or laminate moves through the flow wrapping machine. The dosage units are introduced into the packaging line by a precise dosing or feeding mechanism, which deposits one unit at a time onto the moving film.

The feeding system is synchronized with the movement of the film to ensure that each solid dosage unit is positioned correctly on the centerline of the tubular packaging material, enabling uniform sealing and minimal material waste. The dosage units are spaced at regular intervals to match the desired final package size.

In step d) the primary packaging film or laminate, now formed into a tubular shape with individual solid dosage units, is sealed around each unit to create a hermetic seal. This seal is achieved through the application of heat and/or pressure to the film or laminate. The sealing process typically involves two stages:
- Longitudinal Sealing: The overlapping edges of the tubular film are sealed together along the length of the package. This is done by passing the film through a set of heated sealing rollers or a heat bar that applies controlled pressure and temperature to fuse the edges of the compostable film or laminate without damaging its structure or integrity.
- Transverse Sealing: After the longitudinal seal is completed, the tubular film is sealed transversely between each solid dosage unit. This creates individual sealed compartments around each dosage unit. Heat-sealing jaws apply heat and pressure at the points between the dosage units, ensuring an airtight and moisture-resistant barrier around each unit. This prevents contamination, degradation, and exposure to environmental factors.

The temperature and pressure applied during the sealing process are carefully controlled to match the characteristics of the primary packaging film or laminate, ensuring a strong seal without compromising the compostable nature of the packaging.

In an optional step e) the continuous tubular film is cut into individual packages. A cutting mechanism, typically a rotary knife or blade, is positioned downstream of the transverse sealing station to sever the tubular film at the sealed areas, creating individual packages. Each package contains a single solid dosage unit hermetically sealed within the compostable primary packaging. The cutting is synchronized with the sealing process to ensure that the film is cut precisely at the sealed areas, avoiding damage to the sealed compartments or the contents inside.

In an optional step f) the individually wrapped solid dosage units are collected for further handling. The flow wrapping machine may be equipped with a conveyor system or collection tray to receive the finished packages. The individually wrapped units are then ready for storage, distribution, or further packaging (e.g., secondary packaging like cartons or boxes).

The method may accommodate different sizes and shapes of solid dosage units, allowing flexibility in packaging various medicinal products using the same compostable film or laminate.

The process may further include quality control checks such as automated weight checks, visual inspections, or other non-destructive testing methods to ensure that each package is correctly sealed and contains the intended solid dosage unit.

In some embodiments the method further comprises the providing the wrapped individual solid dosage units with information concerning the medicinal product. Labeling or coding can be integrated into the process to add batch numbers, expiration dates, or barcodes to the film before or after the sealing process. This can be done via thermal printing or inkjet systems. The primary packaging must typically include specific information to ensure proper identification, safe use, and traceability of the product. This is regulated by health authorities such as the European Medicines Agency (EMA), the U.S. Food and Drug Administration (FDA), and other national regulatory bodies. The exact requirements can vary slightly by jurisdiction, but generally, the information should include the name of the medicinal product, which can include both the brand name and the generic (International Non-proprietary Name, INN) or scientific name, the name of the active ingredient, if necessary, the strength of the product (e.g., 500 mg), the pharmaceutical form (e.g., tablet), batch number (lot number), expiry date (typically in the format of month/year, e.g., Exp: 08/2024), route of administration, storage conditions, special warnings or precautions, marketing authorization holder, name and address of the company or entity responsible for the product's marketing authorization, regulatory approval number, and barcodes or serialization. However, for very small packaging regulatory agencies may allow some of the above information to be omitted, provided it is available on the outer packaging. Thus, on the wrapped individual solid dosage units of the present disclosure it may be sufficient to provide the name of the medicinal product, the name of the active ingredient, if necessary, the strength of the product (e.g., 500 mg), the pharmaceutical form (e.g., tablet), batch number (lot number), expiry date (typically in the format of month/year, e.g., Exp: 08/2024), and the name of the company or entity responsible for the product's marketing authorization.

According to a third aspect illustrated herein, there is provided an individual solid dosage unit of a medicinal product wrapped and hermetically sealed in a compostable primary packaging as described above with reference to the first aspect. The compostable primary packaging and the wrapped solid dosage unit of a medicinal product may be further defined as described above with reference to the first aspect.

The wrapped solid dosage unit of a medicinal product of the present disclosure may preferably be designed in a way that prevents unauthorized access to the medicinal product. In some embodiments, the wrapped solid dosage unit of a medicinal product of the present disclosure is configured to be tamper proof. In the context of medical packaging, tamper-proof refers to packaging that is designed to prevent unauthorized access or tampering with the contents, providing a visible indication if any attempt has been made to interfere with the product. This type of packaging ensures the safety, integrity, and authenticity of the medicinal product, protecting it from contamination, alteration, or theft. Tamper-proof packaging is essential in maintaining patient safety and public trust in pharmaceuticals.

In some embodiments, the wrapped solid dosage unit of a medicinal product of the present disclosure is rendered tamper proof by a high sealing strength, such that the sealed portions of the packaging cannot be opened, without causing significant and visible damage to the primary packaging film or laminate.

The medicinal product of the present disclosure may be any medicinal product provided in solid form. However, it has been found that the primary packaging and methods for wrapping of the present disclosure may be especially advantageous for ethical prescription medicinal products. Ethical prescription medicinal products refer to medicinal products that can only be dispensed to patients with a valid prescription from a licensed healthcare provider, such as a doctor, dentist, or nurse practitioner. The term "ethical" in this context relates to the ethical responsibility of healthcare professionals to determine when a drug should be prescribed, rather than the drug being available for direct consumer purchase.

Generally, while the products, polymers, materials, layers and processes are described in terms of "comprising" various components or steps, the products, polymers, materials, layers and processes can also "consist essentially of" or "consist of" the various components and steps.

While the invention has been described with reference to various exemplary embodiments, it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiment disclosed as the best mode contemplated for carrying out this invention, but that the invention will include all embodiments falling within the scope of the appended claims.

## Claims

1. A compostable primary packaging suitable for wrapping individual solid dosage units of a medicinal product, comprising:
a primary packaging film or laminate comprising a biodegradable and
compostable polymer, the film or laminate being configured to be processed in a film wrapping machine to encase an individual solid dosage unit of a medicinal product;
the primary packaging film or laminate having sufficient mechanical strength, flexibility, and sealability to maintain an airtight and moisture-resistant barrier around the solid dosage unit after a film wrapping process;
the primary packaging film or laminate being **characterized by** its ability to fully decompose into non-toxic organic components within an industrial composting environment within a typical composting cycle of 90 days.

2. The compostable primary packaging of claim 1, wherein the primary packaging film or laminate is **characterized by** its ability to fully decompose into non-toxic organic components within an industrial composting environment within a typical composting cycle of 90 days, in accordance with European Standard EN 13432, American Standard ASTM D6400, or International Standard ISO 17088.

3. The compostable primary packaging of any one of the preceding claims, wherein the biodegradable and compostable polymer is selected from the group consisting of polylactic acid (PLA), polybutylene succinate (PBS), polyhydroxyalkanoates (PHA), polycaprolactone (PCL), thermoplastic starch (TPS), cellulose-based polymers, chitosan, alginates, or a combination thereof.

4. The compostable primary packaging of any one of the preceding claims, wherein the biodegradable and compostable polymer is selected from the group consisting of polylactic acid (PLA), polybutylene succinate (PBS), polyhydroxyalkanoates (PHA), polycaprolactone (PCL), thermoplastic starch (TPS), or a combination thereof.

5. The compostable primary packaging of any one of the preceding claims, wherein the biodegradable and compostable polymer comprises an algae-based polymer.

6. The compostable primary packaging of any one of the preceding claims, wherein the primary packaging film or laminate comprises a laminate, wherein at least one layer is composed of a biodegradable and compostable polymer selected from the group consisting of polylactic acid (PLA), polybutylene succinate (PBS), polyhydroxyalkanoates (PHA), polycaprolactone (PCL), thermoplastic starch (TPS), or a combination thereof.

7. The compostable primary packaging of claim 6, wherein the laminate comprises a first layer composed of polylactic acid (PLA) to provide strength and rigidity, and a second layer composed of polybutylene succinate (PBS) to provide flexibility and sealability.

8. The compostable primary packaging of claim 6, wherein the laminate comprises an inner layer composed of polyhydroxyalkanoate (PHA) for direct contact with the medicinal product, and an outer layer composed of a thermoplastic starch (TPS) for improved barrier properties against moisture and oxygen.

9. The compostable primary packaging of claim 6, wherein the laminate comprises a layer of polycaprolactone (PCL) to provide heat-sealability, and a layer of polylactic acid (PLA) to provide strength and rigidity.

10. The compostable primary packaging of any one of the preceding claims, wherein the primary packaging film or laminate comprises an outer surface treatment or coating to enhance barrier properties against oxygen and moisture.

11. The compostable primary packaging of any one of the preceding claims, wherein the primary packaging film or laminate comprises a thin-film coating layer to enhance barrier properties against oxygen and moisture.

12. The compostable primary packaging of any one of the preceding claims, wherein the packaging film or laminate is free from non-compostable additives.

13. A method for wrapping individual solid dosage units of a medicinal product using a compostable primary packaging film or laminate, comprising the steps of:
a) providing a compostable primary packaging comprising a primary packaging film or laminate according to any one of the preceding claims;
b) feeding the primary packaging film or laminate into a film wrapping machine, wherein the film or laminate is configured to be formed around the individual solid dosage units of the medicinal product;
c) placing individual solid dosage units of the medicinal product onto the primary packaging film or laminate as it moves through the film wrapping machine; and
d) sealing the film or laminate around each individual solid dosage unit by applying heat and/or pressure to create a hermetic seal, thereby ensuring an airtight and moisture-resistant barrier.

14. The method of claim 13, comprising the steps of:
a) providing a compostable primary packaging comprising a primary packaging film or laminate according to any one of claims 1-13;
b) feeding the primary packaging film or laminate into a flow wrapping machine, wherein the film or laminate is configured to be formed into a continuous tubular shape around the individual solid dosage units of the medicinal product;
c) placing individual solid dosage units of the medicinal product onto the primary packaging film or laminate as it moves through the flow wrapping machine;
d) sealing the film or laminate around each individual solid dosage unit by applying heat and/or pressure to create a hermetic seal, thereby ensuring an airtight and moisture-resistant barrier; and optionally
e) cutting the continuous tubular film into individual packages, each containing an individual solid dosage unit of the medicinal product; and
f) collecting the individually wrapped solid dosage units for further handling, storage, or distribution.

15. An individual solid dosage unit of a medicinal product wrapped and hermetically sealed in a compostable primary packaging according to any one of the preceding claims.
